# EUROPEAN PATENT APPLICATION

(11) **EP 1 767 617 A1**
(43) Date of publication of application: **28.03.2007**
(21) Application number: 05425670.6
(22) Date of filing: 26.09.2005
(51) Int. Cl.: C12N 5/06

(54) **Mesenchymal stem cells isolation and expansion method and uses thereof**

(71) Applicant: Mazzini, Letizia, 28100 Novara (IT); Fagioli, Franca, 10127 Torino (IT)
(72) Inventor: Mazzini, Letizia, 28100 Novara (IT); Fagioli, Franca, 10127 Torino (IT)
(74) Representative: Freyria Fava, Cristina

(57) **Abstract**

The present invention relates a mesenchymal stem cells isolation and expansion method comprising the steps of (i) isolating the mesenchymal stem cells from a sample of biological tissue, and (ii) exposing the mesenchymal stem cells to such conditions to allow the espansion of said mesenchymal cells, where the step (i) provides the use of a density gradient based on a sucrose polymer to isolate the cells from the tissue. The present invention further concerns the use of mesenchymal stem cells so obtained in clinical field for treating neurovegetative pathologies.

## Description

### Field of the invention

The present invention relates a mesenchymal stem cells isolation and expansion method and the clinical use of said cells in patients affected by neurodegenerative pathologies.

### Technical background of the invention

Medullary mesenchymal stem cells (MSC) are easily isolable and expandable and are able to differentiate in different cell lines of mesenchymal type: osteoblasts, chondrocytes, adipocytes, myocytes, tenocytes and stromal cells (Pittenger MF 1999). Moreover, they are able to overcome their germinal commissioning since studies, both in vitro (Sanchez-Ramos J 2000; Woodbury D 2000; Woodbury D 2002, Deng et al. 2001, Kim BJ 2002, Terada N.. 2002, Jang Y. 2002) and in vivo (Kopen GC 1999, Zhao LR 2002, Azizi SA 1999, Jin HK 2002 J, Hofstetter CP 2002) demonstrate their differentiation also in neuronal sense.

In many grown-up tissues, cell loss is balanced by proliferation and differentiation of stem cells. Recent experimental observations suggest that neurodegenerative pathologies can involve alterations in proliferation, migration and differentiation of stem cells. A growing number of studies carried out on animal models show how stem cells have the ability to specifically migrate in areas in which a tissular damage has been induced and to proliferate and differentiate in neurons and glial cells. These observations demonstrate that MSCs are able to overcome their specific commissioning in mesodermic sense and make them ideal candidates for tissular regeneration. Further studies in several experimental models demonstrate that, once implanted at the level of the central nervous system, they are able to take root, differentiate in neural sense and regenerate the nervous tissue at the site of damage (Hofstetter CP 2002, Sasaski M 2001, Mahmood A 2001, Chopp M 2002, Jin HK 2002). Medullary stem cells transplanted in different animal models of nervous central system diseases have demonstrated to possess positive clinical effetcs. MSCs have induced an improvement in neurological deficiencies in rats with cerebral ischemia and have postponed the onset of neurological abnormalities and prolonged the life in mice with sphingomyelinic acid deficiencies.

These observations lay the bases for a potential clinical exploitation of MSCs in neurodegenerative pathologies as Amiotrophic Lateral Sclerosis (SLA), a degenerative disease of the central nervous system that selectively affect motoneurons and that up till now do not have an efficient therapy. Progressive death of motoneurons clinically translates itself in a progressive plegia of the whole spinal and bulbar musculature till an exitus generally determined by respiratory insufficiency after about 3 years from the onset.

Though the matter has roused some controversy about their potential differentiation *in vivo* in neurons and glia (Castro et al., 2002; Wagers et al., 2002; Alvarez-Dolado et al., 2003; Weimann et al., 2003a), it was observed that in mice models of cerebral infarct, cerebral and spinal trauma and Parkinson disease, MSCs are able to migrate towards damaged tissue and ameliorate the recovery of neurological deficiencies (Li et al., 2000; Li et al., 2001).

Several clinical applications of MSCs exist. Particularly, promising results have been obtained using human MSCs in controlled clinical studies in patients affected by osteogenesis imperfecta (Horwitz et al., 1999; Horwitz et al., 2001; Horwitz et al., 2002), methachromatic leucodystrophy and Hurley's syndrome (Koc et al., 2002). Moreover, in virtue of their immunosuppressive properties and their ability to support hemopoiesis, MSCs have been used to reduce acute and chronic transplant disease against the host (Lee, 2002, Le Blanc, 2004; Lazarus et al. 2005) and to facilitate hemopoietic rooting after a bone marrow transplant (Koc et al., 2000). Moreover, MSCs differentiated in osteoblasts have been transplanted in "distraction osteogenesis" (Kitoh, 2004) and after engineering in patients affected by ostegenesis imperfecta (Horwitz et al., 2002).

### Summary of the invention

The object of the present invention is to provide a new method of mesenchymal stem cells (MSCs) isolation and expansion that allows to increase yield of MSC cells. Moreover, the present invention concerns the use of mesenchymal stem cells so isolated ed expanded to prepare a medicament for treatment of neurodegenerative pathologies.

A further object of the present invention is to provide an *in vitro* composition comprising mesenchymal stem cells, preferably medullary, isolated and expanded according to the method of the present invention to be used in the clinical field for therapy of neurodegenerative pathologies - with intraspinal or intracerebral implant techniques - reducing microbiological contamination risks of such compositions.

According to the present invention, such objects are achieved in virtue of the solutions specifically recalled in the appended claims. The claims are integral part of the tecnical instructions here provided in connection with the invention.

### Detailed description of the invention

The present invention will be now described in more detail with reference to a currently preferred embodiment through non-limitative examples.

The present solution provide a new method for MSCs isolation and expansion, preferably of medullary origin, allowing to obtain both qualitative and quantitative better results with respect to isolation and expansion methods currently known.

Specifically, a preferred embodiment of the present invention relates a mesenchymal stem cells isolation and expansion method comprising the steps of (i) isolating mesenchymal stem cells from a sample of biological tissue, and (ii) exposing the mesenchymal stem cells to such conditions to allow their espansion, where the isolation step (i) provides the use of a gradient based on a sucrose polymer such as, e.g., the Ficoll (Biochrom KG, DE) preferably at a density of 1.077 g/ml.

The method according to the present invention allows, totally unexpectedly and surprisingly, to obtain a higher cell yield with respect to methods known in the art. The use of a density gradient composed of a sucrose polymer in contrast to the use of a density gradient based on colloidal silica, such as Percoll at a density of 1.073 g/ml as described in Mazzini et al. 2003, has actually allowed not only to increase MSC cell yield, that present the same immunophenotipic characteristics of MSCs isolated with colloidal silica, but to obtain MSC cells that have a proliferative potential surprisingly higher with respect to cells isolated from Percoll gradient.

Also, the alteration effected to the vehicle of the in vitro composition comprising MSCs, and particularly the choose of a sterile physiological solution, has allowed to reduce/avoid microbiological contaminations with respect to the prior art where - as vehicle - liquor taken from the pazient to be trated was used.

More particularly, the currently preferred embodiment of the present invention relates a mesenchymal stem cells isolation and expansion method comprising the steps of:
(a) washing the tissue sample, preferably, bone marrow, obtained from deplasmated, nucleated cells;
(b) stratifying on gradient base on sucrose polymer, preferably at a density of 1.077 g/ml, nucleated cells obtained from the bone marrow;
(c) centrifugating the nucleated cells previously stratified, so as to obtain mononucleated cells at the interface;
(c1) collecting and washing mononucleated cells at the interface;
(d) inoculating mononucleated cells on a culture medium at a cell density between 500,000/cm² and 1,000,000/cm², preferably, 800,000/cm², allowing the expansion of such cells;
(e) removing from culture medium non-adherent cells;
(e1) refeeding every 3-4 days eliminating a part of culture medium and adding new culture medium;
(f) separating from culture medium mesenchymal stem cells adherent at confluence, preferably after about 15 days, by a solution containing trypsine/EDTA, to obtain separated mesenchymal stem cells;
(f1) adding a solution trypsine-nautralizing and recovering the cells separated from culture medium;
(f2) centrifugating mesenchymal stem cells separated and cellular counting;
(g) inoculating mesenchymal stem cells so separated in cell culture flasks at a cell density between 5,000/cm² and 10,000/cm², preferably 8,000/cm², allowing the expansion of such cells.

The expansion phase of mesenchymal stem cells is conducted by repeating the steps (f) to (g), preferably every week, until reaching of the number of mesenchymal stem cells required.

The biological tissue employed for isolating the mesenchymal stem cells is selected from bone marrow, cordal blood, mobilized peripheral blood, adipose and fetal tissue, amniotic liquor, muscle, brain and teeth. In a currently preferred embodiment, MSCs are isolated from bone marrow because easily available from collection of medullary blood obtained in local anaesthesia according to a very long time consolidated standard method. Moreover, from this source, MSCs are easily isolable and expandable without any cell manipulation as immunogenetic separation or a treatment with grow factors.

In a currently preferred embodiment, the method according to the present invention provide the use of a discontinuous density gradient based on a sucrose polymer with molecular weight of between 70 and 400,000 Da, preferably 400,000 Da, and with a density of between 1.076 and 1.078 g/mL, preferably 1.077 g/mL.

### In vitro studies

Differentiation of MSC in neuronal-like cells expressing neuronal markers was reported both *in vitro* and *in vivo* after a trasplant in brain and spinal cord (Sanchez-Ramos et al., 2000; Kopen et al., 1999; Deng et al. 2001; Kohyama et al., Kim et al., 2002; Zhao et al., 2002; Hofstetter et al., 2002). To classify the actual neuronal differentiation, the cells must have the biochemical, morphological and electrophysiological characteristics of mature neurons. Some neuronal markers can be used to study the differentiation process. Particularly, Nestine is commonly used as a marker of neural precursors, Neuron Specific Enolase (NSE) (Woodbury et al., 2000) as neural marker increasing during differentiation, Neu-N as neuron-specific marker expressed in post-mitotic cells (Sarnat et al., 1998), MAP-2 as mature neural marker (Long, 2005) e GFAP as astrocyte marker (Eng, 1971). Some molecules as cytokines, growth factors, neurotrophines, retinic acid, antioxidant demethylating agents, compounds that increase intracellular cAMP and physiological neural inductors (Sanchez-Ramos et al., 2000; Takahashi et al., 1999; Jiamg et al., 2003; Kabos et al., 2002; Koyama et al., 2001), are used to induce neural differentiation in vitro of mice and human MSCs. Lu et al. have demonstrated that morphological changes of rat MSCs and an increase in immunoreacivity for some neuronal markers by chemical induction in culture are probably the result of cellular toxicity, cellular restriction and structural changes of cytoskeleton and do not rappresent stages of the cell differentiation process in neural sense (Lu et al., 2004). Moreover, though MSCs in specific conditions are able to express proteins identified as neural markers at different stages of neuronal development, it is not evident if these neuronal-like cells have the functional activity of true neurons.

The present inventors have treated MSCs with four different experimental conditions to induce neuronal differentiation, respectively:
1) α-MEM + beta-mercaptoethanol for 24 hours;
2) α-MEM + Dimetilsulphoxide (DMSO) + beta-hydroxy-anisole (BHA) for 24 hours;
3) α-MEM + trans-retinoic acid (ATRA) for 72 hours;
4) Neural Progenitor Maintenance Mediun (NPMM) for at least 2 weeks.

Non-treated MSCs appeared with a fibroblastic oblong shape and immunocytochemical analyses showed negativity for GFAP, NSE and NeuN and a low positivity for Nestine and MAP2. With beta-mercaptoethanol, after few hours MSCs changed morphology, showing a neuronal-like appearance with coarctated bodies and extensive prolongations. Analyses by immunofluorescence showed differentiated cells positive for Neu-N, NSE and GFAP. The cells positive for NSE co-expressed also GAFP. Nestine showed a low positivity in differentiated cells thad co-expressed GFAP, where the cells positive for MAP2 co-expressed NSE. In experimental conditions respectively with ATRA and DMSO + BHA, MSCs showed a change in morphology and positivity for NeuN. Instead, these cells were negative for GFAP, Nestine, NSE and MAP-2. In two experiments, expressione of Neu-N not only was nuclear, but also cytoplasmatic, as reported by Lind. MSCs in culture with NPMM slowly assumed a neuronal morphology and, during the treatment, decreased in number after 2-3 days and showed a neuronal-like morpholopgy with cellular prolongations extended after 2-3 weeks. After 2 weeks, the differentiated cells with NPMM were positive for GFAP, NSE and negative for Neu-N, Nestine and MAP2. The cells positive for NSE co-expressed GFAP and immunocytochimic analysis do not changed after 3 weeks. In the studies of RT-PCR, MSCs expressed the trascripts for MAP2, NSE and NF-M. The GFAP mRNA was not detectable in any of conditions of differentiation induction, as in conditions of non-differentiation. The most relevant data in this study were obtained by electrophysiological studies that demonstrated the presence of currents with respect to the K⁺ heag1 and heag2 channels in the cells differentiated in NPMM. Such channels were not present in non-differentiated MSCs and are identified as channels specifically expressed in neuronal cells.

### In vivo studies

Recently, the present inventors have utilised a transgenic mice model for the superoxyde dismutase-1 gene (SOD1), that is considered an experimental model of neurodegenerative pathology SLA, though not exactly reproducing the characteristic of the human disease and its progression. Indeed, in transgenic mice SOD1, motoneuronal damage is induced by a genetic mechanism that is not the etiological mechanism common in human SLA. The animal model is likewise useful to study the action mechanism of stem cells transplanted and the method security, but less useful as indicator of clinical effects of the treatment effected on patients affected by SLA. As showed in many previous clinical trials with neurotrophic growth factors, the evidence of a positive effect meaningful in animal models was not confirmed in human beings.

Without being bound to any particular theory, the present inventors have good reasons to believe that MSCs transplanted in SLA patients could act through different mechanisms; for example, it could be assumed that mesenchymal stem cells in SLA can determine production of cytokines and neurotrophic factors, motoneuronal reflected activation, motoneuronal regeneration, instead of neuron substitution, which can be clinically translated in a slowing down of disease progression.

Human embryonic stem cells have generated neurons when transplanted in spinal cord of a SOD1 mouse (Maragakis, 2000). Moreover, it was demonstrated that SOD1 mice transplanted intravenously after irradiation (800 cGy) with a high dose of mononucleated cells isolated from human umbilical cord survived for a more long time with respect to non-transplanted control mice (Ende, 2000). This effect was meaningfully enhanced by doubling the implanted cells dose (Chen, 2000). Moreover, the transplant of stem cell derived from a human teratocarcinoma line in spinal cord of a SOD1 mouse slowed disease progression (Garbuzova-Davis, 2001) and prolonged survival (Garbuzova-Davis, 2002). Also using a different animal model of SLA, a Sindblis virus model, it was demonstered that injection of embryonal stem cells in spinal fluid have improved muscular strenght of paralysed rats (Vastag, 2001).

### EXAMPLES

### Example 1 - Isolation and expansion of mesenchymal cells

The isolation and expansion protocol of mesenchymal stem cells presented here is in accordace with "Linee guida per l'avvio degli studi clinici di fase I/II con cellule umane viventi per la terapia cellulare somatica" (Notiziario ISS Vol. 10, n. 5 1997), "Linee guida per l'ingegneria dei tessuti e la terapia cellulare" (Notiziario ISS Vol. 12, n. 5 1999) dell'Istituto superiore di Sanità and ISTSAN Report 01/22, 2001.

Explanted bone marrow is washed with PBS 1X at 900g for 10 minutes. The cells are stratified on Ficoll gradient (density: 1.077 g/ml) and centrifugated at 1000 g for 30 minutes. The cells present at the interface are recovered and washed 2 times with PBS 1X (800g for 10 minutes). The cells so obtained are inoculated at a density of 800.000/cm² in *MSC Medium* (Cambrex, UK) in 150 cm² flasks and maintained at 37 °C with an atmosphere od CO₂ 5%. After 3 days, the non-adhaerent cells are removed and refeeds are effected every 3-4 days. After 15 days, the cells are detached with Trypsine/EDTA for 5 minutes at 37 °C, and thereafter a Trypsin-neutralizing solution is added. The detached cells are counted and again plated at 8,000 cells/cm² in new 150 cm² flasks. The following steps are effected every 7 days with the same method since obtainment of the requested cell number for the transplant (in total, 2-4 weeks).

The expanded cells are precisely characterized and analyzed at every step of the expansion. The following test are carried out on cells:
- Evaluation of sterility (the absence of contamination of anaerobic, aerobic bacteria and fungi) at every medium change (every 3-4 days), and at every passage by routine bacteriological analysis on 10 ml of cellular supernatant;
- Evaluation of the absence of contamination by mycoplasma by analysis in Polymerase Chain Reaction (PCR) on 300 µl of cellular supernatant;
- Evaluation of the absence of contamination by bacterial endotoxins by kinetic chromogenic test (Kinetic-QCL, Camrex, UK) on 100 µl of cellular supernatant that are incubated for 60 minutes at 37 °C in precence of Limulus Amebocyte Lysate. The reading is effected at 405-410 nm. The test has a sensibility range from 50 EU/ml to 0,005 EU/ml.
- Evaluation of cell viability by analysis with Trypan Blue (the cells are observed with an optical microscope in a Burker chamber) and by immunocytofluorimetric analysis without propidium chloride. For such analysis, 200,000 cells are incubated for 10 minutes with 200 µl of a solution containing 0,5 µg/ml of propidium iodide in PBS 1X and evaluation is made on cells negative to propidium iodide.
- Immunological determination of markers that characterize mesenchymal stem cells (CD45, CD14, CD90, CD106, CD29, CD44, CD166, CD105) by cytofluorimetric analysis. Such analysis is effected on 500,000 cells that are incubated 20 minutes at 4 °C in the dark with antibodies marked with Fluorescein Isothiocyanate (FITC) or Phycoerithrine (PE).
- Cytogenetic examination to exclude alteration acquired from chromosome complement (karyotype analysis). For karyotype analysis at least 25 metaphases are analyzed starting from 200,000 cells in culture at every stage.
- Measurement of cell senescence by analysis of telomer length in Flow-FISH by cytofluorimetric analysis on 500,000 cells that have been freezed in liquid nitrogen at the time of the explantation (on cell populations CD34+ and CD34- selected by immunomagnetic antibodies) and at every stage of expansion.

### Example 2 - Comparison of protocols of isolation of MSC cells according to the prior art and according to the present invention

Normally, human MSCs of bone marrow are isolated from the portion of mononucleated cells, that can be separated from residual cellular component by stratifying on discontinuous density gradient.

Isolation and expansion protocol described in Mazzini et al., 2003, provided the use of a gradient based on colloidal silica, marketed with the trade name Percoll (Sigma, USA), at a density of 1.073 g/mL. Percoll at this density must be prepared from a mother solution with a density of 1.130 g/mL and preparation at the desired density need the addition of NaCl 1,5M and distilled water. 1,5M NaCl solution must be prepared from solute and thereafter autoclaved. Percoll at the desired density must then be tested for osmolarity, pH and sterility.

In Table 1 are resumed result characteristics using the gradient according to the prior art (Percoll) and the gradient according to the present invention (Ficoll - Biochrom KG, De) and a comparison of the results on MSCs isolation with the two gradients. Particularly, immunophenotype by cytofluorimetric analysis and the growth curve of the cells by cumulative cell duoplication (cumulative PD) were analyzed after 15 passages (about 100 days in culture).

**Table 1.**

| | PERCOLL Prior art | FICOLL Present invention |
|---|---|---|
| Gradient density | 1.073 g/mL | 1.077 g/mL |
| Preparation | With NaCl 1,5 M and H₂O H₂O | Ready for use |
| pH and osmolarity | To be controlled | Estabilished at the time of use |
| Sterility | To be controlled | Certified |
| MNC recovery after centrifugation | 20.5 ±6.1% (n=8) | 29.9 ±9.8% (n=11) |
| Cytofluorimetric analysis of MSC | CD45-, CD34-, CD90+, CD105+, CD29+, CD44, CD166+, CD106+ | CD45-, CD34-, CD90+, CD105+, CD29+, CD44, CD166+, CD106+ |
| Cumulative PD obtained after about 100 days in culture | 10.3 ±2.0 (n=8) | 18.0 ±2.0 |
| Differentiation | In adipocytes, chondrocytes, osteoblasts, neural cells | In adipocytes, chondrocytes, osteoblasts, neural cells |

On 8 samples of bone marrow analyzed, the average of mononucleated cells isolated by Percoll was 20.5 ±6,1% of total cells, while analyzing 11 samples of bone marrow stratified on Ficoll gradient, the percent of recovery of mononucleated cells increased to nearly 10%. Because MSCs are isolated from a portion of mononucleated cells it is assumable that the number of MSCs isolated with a separation system of Ficoll gradient is increased.

Moreover, MSCs obtained by Ficoll show the same immunophenotypic characteristics of MSCs isolated by Percoll, but with a higher proliferative potential. Particularly, analyzing the cell growth of MSCs isolated with the two gradients through the study of cumulative PD it was observed that MSCs isolated from Ficoll gradient show a proliferative potential (18.0) surprisinlgy higher with respect to those isolated from Percoll gradient (10.3).

The chosing to use Ficoll gradient instead of Percoll has thus allowed to improve starting cell yields to obtain a higher number of MSCs and to obtain cells with a higher proliferative potential.

### Example 3 - Preparation of mesenchymal stem cells for inoculation

At least three hours before infusion, culture medium with serum is removed and the cells are washed three times with 10 ml of sterile PBS 1X. After the three washings, 10 ml of fresh medium without serum are added in every flask and the flaks are ri-placed in the incubator for one hour to allow the cells to eliminate proteins contained in the serum. Once the medium removed, the cells are washed with PBS 1X and detached with trypsin/EDTA for 5 minutes at 37 °C. Trypsin action is stopped by adding a trypsin-neutralizing solution (TNS). Le cells are harvested and centrifugated at 200g for 10 minutes and thereafter re-suspended in 10 ml of PBS 1X + human albumin (HSA) 1%. An aliquot of cell suspension is collected for cell count while other 10 ml of PBS 1X + 1% HSA are added to cell suspension that is again centrifugated at 200 g for 10 minutes. The cell pellet is re-suspended and washed 2 times again with PBS 1X + 1% HSA.

After the final washing, the cells are re-suspended in about 1 ml of physiological solution and are implanted at the level of the spinal cord after laminaectomia.

The laboratory quality system implemented according to the requirements of rule ISO 9001:2000 verify the total trackability of every patient from the time of introducing of bone marrow pouch to the time of delivery of mesenchymal stem cells.

One proceed to sample preparation for implant and release of cell product for clinical use only if after the entire expansion process the quality test results are compliant results.

The composition is then directly implanted in the spinal cord or at the brain level.

The innovative chosing to transplanting stem cells directly in the spinal cord or at the brain level allows to the stem cells to pass the integral hemato-encephalic barrier as in the case of SLA (Terada N 2002). In fact, in all experimental models used, the stem cells both embrionic and from human teratocarcynoma were directly injected at the intraparenchimal level (Maragakis NJ 2001, Garbuzova-Davis S 2002) or delivered after having damaged the hemato-encephalic barrier (Weistein F 2000). Moreover, no clinical effect was observed in patients with ALS in which the cells were injected in cerebrospinal liquor (Janson CG 2001).

Needless to say, the embodiment details and the embodiments can be largely changed with respect to what described and illustrated above without departing from the scope of protection of the present invention, as determined by the appended claims.

## Claims

1. Mesenchymal stem cells isolation and expansion method comprising the steps of (i) isolating said mesenchymal stem cells from a sample of biological tissue, and (ii) exposing the said mesenchymal stem cells to such conditions to allow the espansion of said mesenchymal cells, **characterized in that** said step (i) provides the use of a gradient based on a sucrose polymer to isolate said cells from said tissue.

2. Method according to claim 1, **characterized in that** said gradient is discontinuous.

3. Method according to anyone of preceeding claims, **characterized in that** said gradient based on a sucrose polymer has a molecular weight between 70 and 400,000 Da, preferably 400,000 Da.

4. Method according to anyone of proceeding claims, **characterized in that** said gradient based on a sucrose polymer has' a density between 1.076 and 1.078 g/mL, preferably 1.077 g/mL.

5. Method according to anyone of preceeding claims, **characterized in that** the step (i) provide a step (a) of washing the said tissue, to obtain nucleated cells.

6. Method according to claim 5, **characterized in that** the step (i) provide the step (b) of stratifying on said gradient said nucleated cells obtained from said tissue, to obtain stratified nucleated cells.

7. Method according to claim 6, **characterized in that** the step (i) further provide the step (c) of centrifugation of said stratified nucleated cells, to obtain mononucleated cells at the interface.

8. Method according to claim 7, **characterized in that** the step (c) further provide the step (c1) of collecting and washing of the mononucleated cells at the interface.

9. Method according to claim 7 or claim 8,
**characterized in that** the step (ii) provide the step of (d) inoculation of said mononucleated cells recovered at the interface on a culture medium, allowing the expansion of said mesenchymal stem cells on said culture medium.

10. Method according to claim 9, **characterized in that** said step of (d) inoculation provide a cell density on said culture medium between 500,000/cm² and 1,000,000/cm², preferably 800,000/cm².

11. Method according to claim 9, **characterized in that** said step (ii) provide the step (e) of removal of non-adhaerent cell from said culture medium, to obtain mesenchymal stem cells adhaerent on said culture medium.

12. Method according to claim 11, **characterized in that** the step (e) further provide the step (e1) of refeeding every 3-4 days and eliminating a part of culture medium and adding new culture medium.

13. Method according to claim 11 or claim 12, **characterized in that** the step (ii) subsequently provide the step (f) of detachment from said culture medium of said mesenchymal stem cells adhaerent at the confluence, preferably after about 15 days, to obtain mesenchymal stem cells detached.

14. Method according to claim 13, **characterized in that** said step (f) of detachment is accomplished using a solution containing trypsine/EDTA.

15. Method according to claim 14, **characterized in that** the step (f) further provide the step (f1) of adding a trypsin-neutralizing solution and recovery of the cell detached from culture medium.

16. Method according to claim 15, **characterized in that** the step (f) further provide the step (f2) of centrifugating the cells detached and cell counting.

17. Method according to anyone of claims 13 to 16, **characterized in that** the step (ii) provide the step (g) of inoculating said detached mesenchymal stem cells in flasks for cell culture, allowing the expansion of said mesenchymal stem cells in said flasks.

18. Method according to claim 17, **characterized in that** the step (g) of inoculating provide a cell density comprised between 5,000/cm² and 10,000/cm², preferably 8,000/cm².

19. Method according to anyone of claims 13 to 16 and according to claims 17 or 18, **characterized in that** the step (ii) provide the repetition of the steps (f) to (g) to the expansion required of said mesenchymal stem cells.

20. Method according to anyone of preceeding claims, **characterized in that** said biological tissue is selected from bone marrow, cordal blood, mobilized peripheral blood, adipose and fetal tissue, amniotic liquor, muscle, brain and teeth.

21. Pharmaceutical composition comprising mesenchymal stem cells obtained with a method according to anyone of claims 1 to 20 and a pharmaceutically accetable carrier.

22. Composition according to claim 21, **characterized in that** said carrier is composed of a sterile physiological solution.

23. Use of mesenchymal stem cells obtained with a method according to anyone of claims 1 to 20 to prepare a medicament for treatment of a neurodegenerative disease in a subject affected from said disease.

24. Use according to claim 23, **characterized in that** said medicament is administered by techniques of intraspinal or intracerebral implant.

25. Use according to claim 23, **characterized in that** said neurodegenerative disease is selected from amiotrophic lateral sclerosis, Parkinson disease, Alzheimer disease and cortico-basal degenerations.

26. Use according to claim 23, **characterized in that** said medicament further comprises a pharmaceutically accettable carrier.

27. Use according to claim 26, **characterized in that** said carrier is composed of a sterile physiological solution.

28. Method of treating a neurodegenerative pathology in a subject affected by such pathology, comprising administering a therapeutically effective amount of mesenchymal stem cells obtained with a method according to anyone of claims 1 to 20 or a composition according to claims 21 or 22.

29. Method of treating according to claim 28, **characterized in that** said mesenchymal stem cells or said composition are administered by techniques of intraspinal or intracerebral implant.

30. Method of treating according to claim 28, **characterized in that** said neurodegenerative pathology is selected from amiotrophic lateral sclerosis, Parkinson disease, Alzheimer disease and cortico-basal degenerations.
